# EUROPEAN PATENT APPLICATION

(11) **EP 4 075 445 A1**
(43) Date of publication of application: **19.10.2022**
(21) Application number: 22168062.2
(22) Date of filing: 13.04.2022
(51) Int. Cl.: G16H 50/30

(54) **DEVICES, SYSTEMS AND PROCESSES TO COMPUTE A VASCULAR HEALTH RELATED SCORE**

(30) Priority: 16.04.2021 US 202117233077
(71) Applicant: Withings, 92130 Issy-les-Moulineaux (FR)
(72) Inventor: VALENCHON, Juliette, 92130 ISSY LES MOULINEAUX (FR)
(74) Representative: Plasseraud IP

(57) **Abstract**

Methods, devices and systems to compute a vascular score for a user, comprising retrieving, from a measuring device, a pulse wave velocity (PWV) related datum of the user retrieving a chronological age datum of the user querying to return a quantile value, computing, using the retrieved chronological age, the returned quantile value and a predetermined law, a vascular score; and generating for display on the measuring device or on a user device, an indication associated with the vascular score.

## Description

### BACKGROUND

. The present disclosure relates to processes, devices, and systems to monitor biomarkers of a human body, in particular biomarkers associated with the cardiovascular system, and the health thereof. The present disclosure proposes processes, devices, and systems to compute a vascular score.

### SUMMARY

. Cardiovascular diseases (CVD) are a major public health problem around the world. Identifying warnings and improving prevention would constitute a steady uphill path towards a better management of CVD.

. The Framingham Heart Study of 2008 (reference [1]) introduced a new a concept of heart age or vascular age, where the *"CVD [cardiovascular risk] of an individual is transformed to an age of a person with the same risk but all other risk factors at the normal lever (nontreated systolic blood pressure of 125 mm Hg, total cholesterol of 180 mg*/*dL, HDL of 45 mg*/*dL, nonsmoker, nondiabetic)"* (see Presentations of page 751). This vascular age is an easy-to-understand form of a score which is computed using a regression model, a log-transformation and several factors such as chronological age, cholesterol, smoker/non-smoker, diabetes, etc. (see Appendix and Table 11). The score is therefore demanding in terms of data to input and calculations to perform.

. More recently, as noted by Bruno *et al.* (reference [2]), pulse wave velocity (PWV) was identified as *"an established marker of early vascular aging"* and *"increased carotid-femoral pulse wave velocity is an established hallmark of arterial stiffening".* This paper proposes to identify a difference between a chronological age and a cardiovascular age, using PWV. More precisely, as disclosed in section *"Results",* vascular age was computed as *"the predicted age in a multivariable regression model, including classical cardiovascular risk factors, treatments and PWV",* where said factors include sex, smoking, height, heart rate, PWV, blood pressure, cholesterol, glycemia, etc. Again, the score is highly complex to determine. Laurent *et al.* (reference [3]) used the extremes of the distribution of vascular aging to define EVA and SUPERNOVA, which stands for Early Vascular Aging and Super Normal Vascular Aging.

. PWV is the velocity at which the blood pressure pulse propagates through the circulatory system. There are different methods to determine a PWV value. PWV corresponds to the distance traveled by the pulse wave divided by the time for the wave to travel the distance. To determine the time, two measures are usually carried out, to obtain a start time and an arrival time.

. A certain number of publications, such as Ferraz-Amaro *et al.* (reference [4]) provided further complements about the relationship between the vascular age and CVD, but they all use a complex formula for the vascular age using regressions.

. Regarding the patent literature, a few publications such as WO2020/239745, US2016029972 (§[0010] for instance), US2019295727 (§[0047] and §[0201] for instance), mention a vascular age and how to compute it but, again, the formula to determine the vascular age is complex.

. The notion of cardiovascular age therefore appears to be a useful score which can easily be understood by the patient. However, many difficulties arise when this type of score needs to be generalized to the whole population (which thus includes users at home and no longer merely patients under medical care). The data needed to compute the score requires professional hardware that only hospitals, institutes, etc. own and many data are required for each individual to compute a single score. In addition, it has been observed that there is no homogenous definition of the vascular age and that most of those definitions involve complex computations that often require a precise PWV value.

. However, PWV is also a difficult measure to get and, as it is not yet widespread in the consumer device world, obtaining a precise value (of the medical level) requires specific tools only available in specialized centers, as explained above. Computing a vascular age with an imprecise PWV value may lead to false conclusions (false positive or false negative for the assessment of CVD risks).

. The present disclosure is aimed at solving at least some of the drawback of the current literature and to enable a quick, easily deployable, and accessible score related to the cardiovascular system health of most of the individuals of the population. This may be achieved using minimal hardware, widely spread across certain countries, such as a weighing device configured to perform a ballistocardiogram BCG (for instance using a ballistocardiography sensor) and an impedance plethysmogram IPG (for instance using an impedance plethysmography sensor). Such hardware includes for instance Withings BodyCardio, which is a personal body scale capable of measuring a BCG and an IPG and therefore to determine a metric pertaining to pulse wave velocity related (referred to as "PWV-related") measures.

. As noted above, computing an absolute vascular age by means of an aggregation of a plethora of data related to an individual, all the data being combined together using complex models, formulas, and regressions, forms a major hurdle that keeps the vascular age metric within the medical world. Instead, the inventors have developed an efficient score that may be computing using a large set of data from a population, without inputting most of the invasive and hard-to-get biometrics data previously cited (blood pressure, diabetes, heart rate, etc.).

. The invention is defined by the appended claims.

. According to an embodiment of the disclosure, it is proposed a method to compute a vascular score for a user, the method comprising: retrieving a pulse wave velocity (PWV) related datum of the user; retrieving a chronological age datum of the user; querying a database using the retrieved PWV-related datum and the chronological age datum to return a quantile value, the database comprising: for a plurality of age groups, a repartition by quantiles of a plurality of PWV-related data and the value of the PWV-related datum associated with each quantile, wherein the returned quantile is the value of the quantile related to the retrieved PWV-related datum; computing, using the retrieved chronological age, the returned quantile value and a predetermined law, a vascular score; generating for display on the measuring device or on a user device, an indication associated with the vascular score (or a vascular score itself).

. According to another embodiment of the disclosure, a method to compute a vascular score for a user, the method comprising: retrieving a pulse wave velocity (PWV) related datum of the user; retrieving a chronological age datum of the user; querying a database using the retrieved PWV-related datum and the chronological age datum to return a Q50 PWV-related datum, the database comprising for a plurality of age groups, a value of the PWV-related datum associated with quantile 0.5, so-called Q50 PWV-related datum; computing, using the retrieved chronological age, the retrieved PWV-related datum, the Q50 PWV-related datum, and a predetermined law, a vascular score; generating for display on the measuring device or on a user device, an indication associated with the vascular score (or the vascular score itself).

. Devices and systems configured to implement the above mentioned methods are also proposed.

### BRIEF DESCRIPTION OF THE DRAWINGS

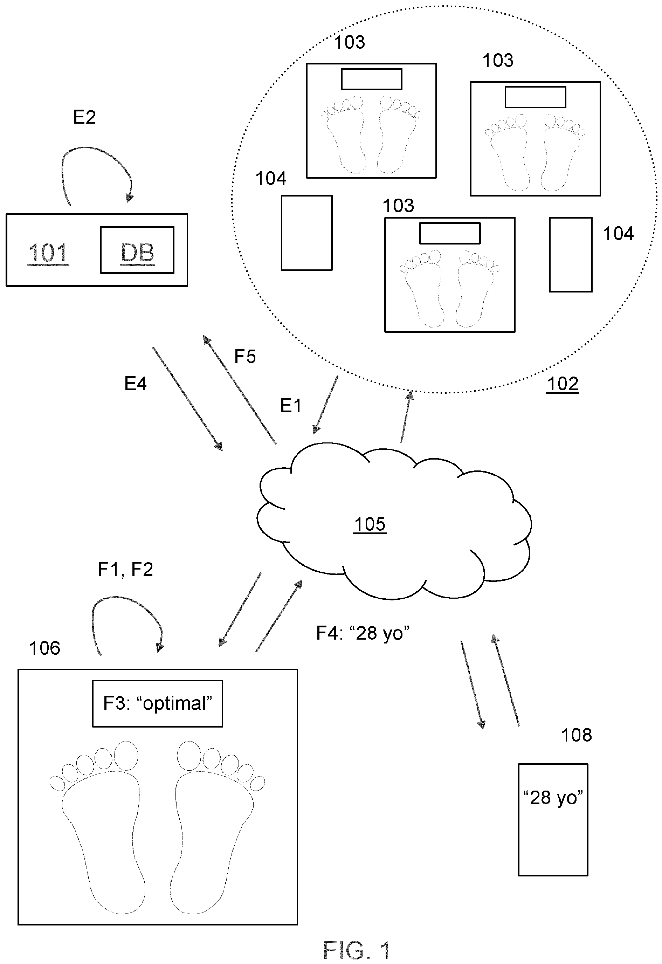

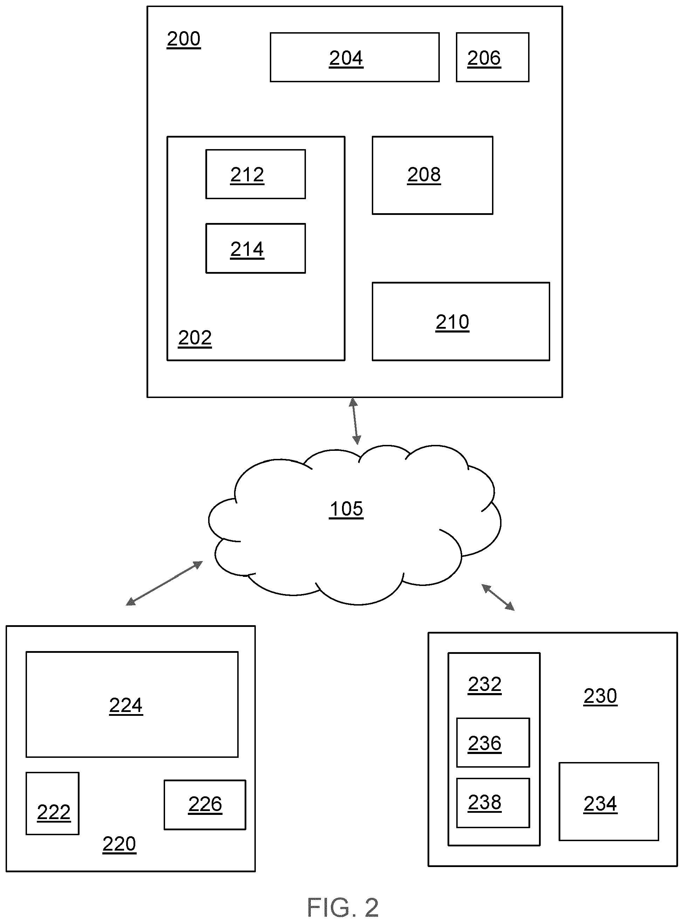

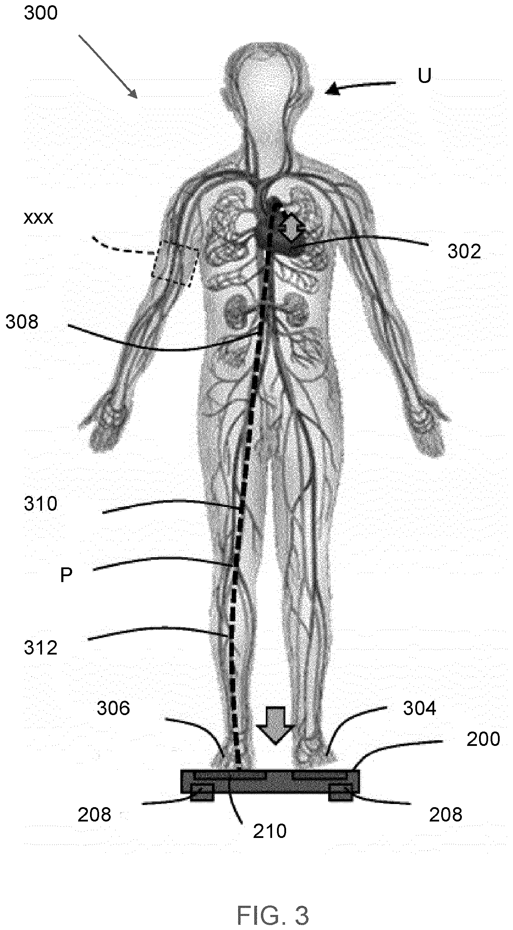

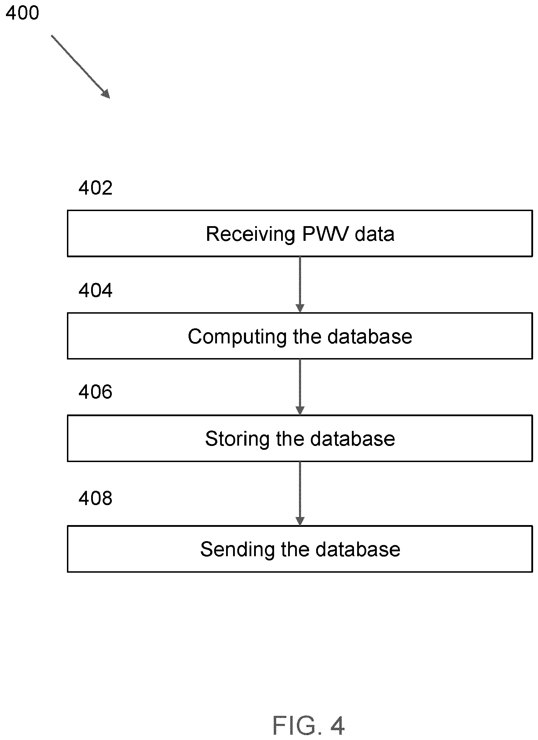

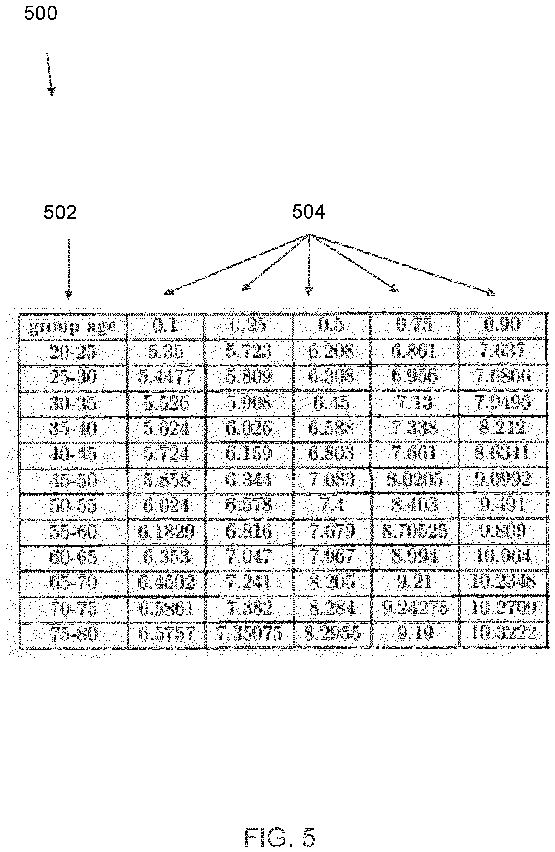

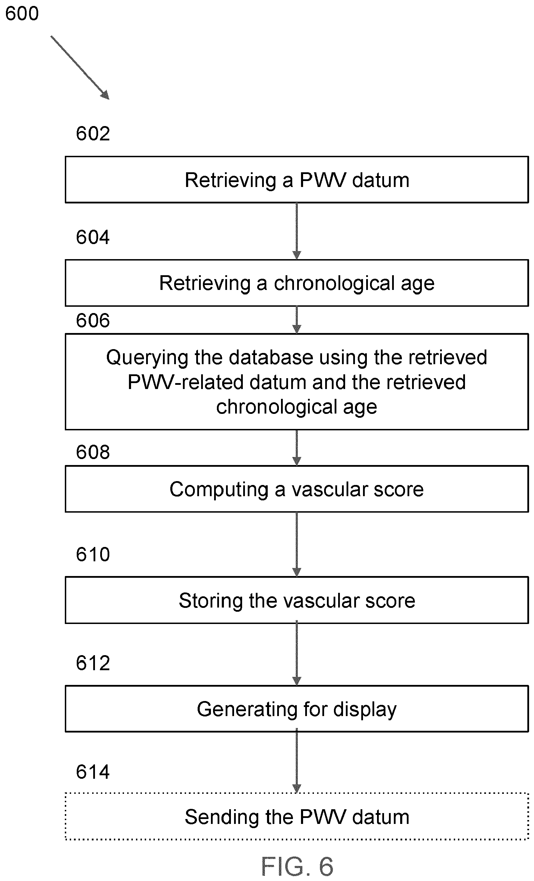

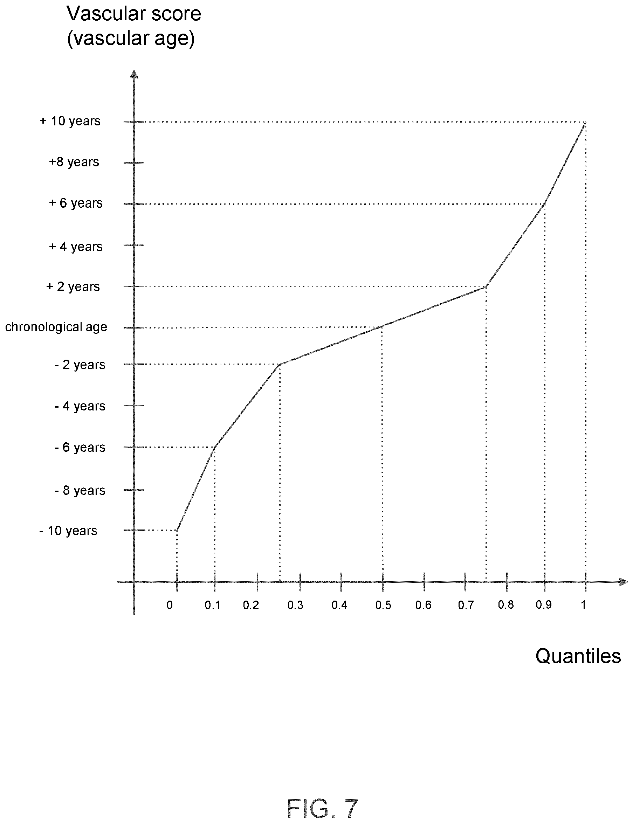

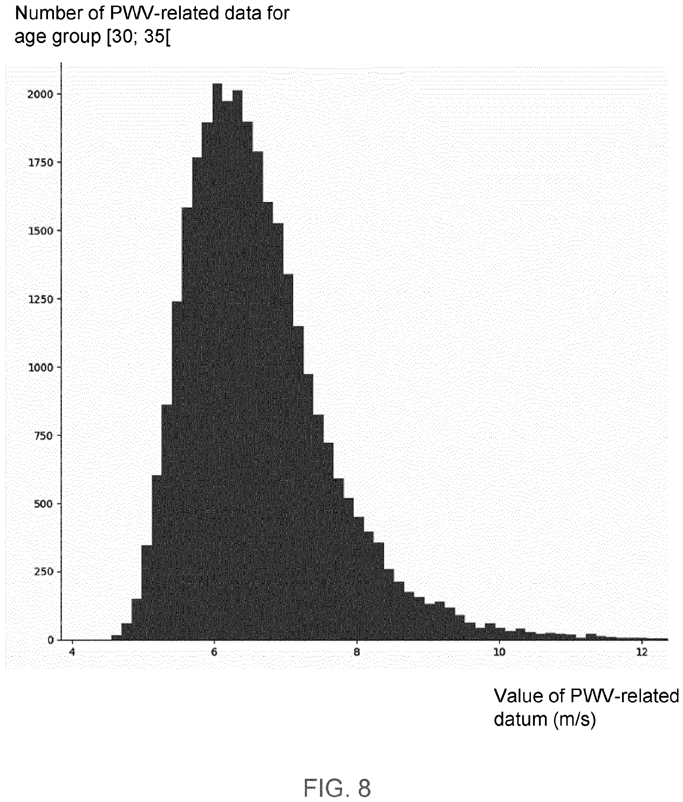

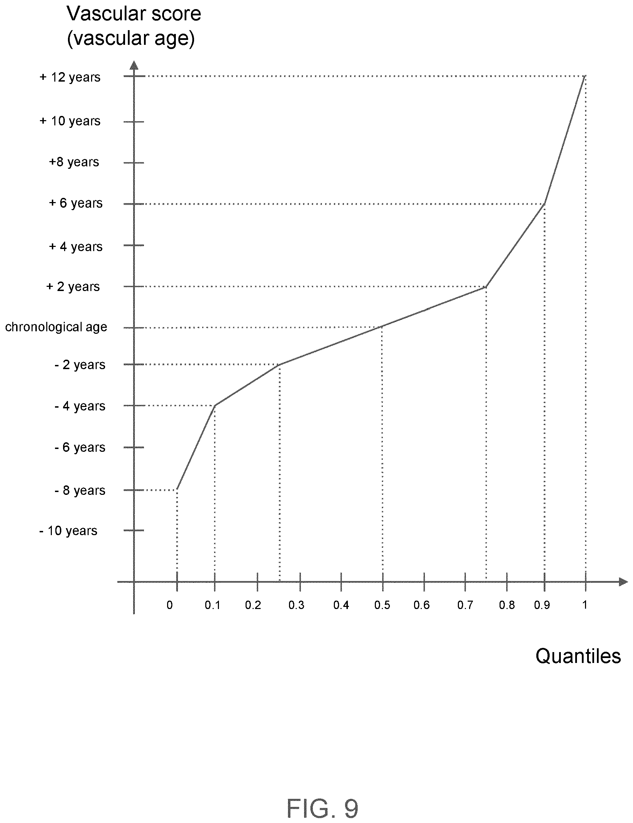

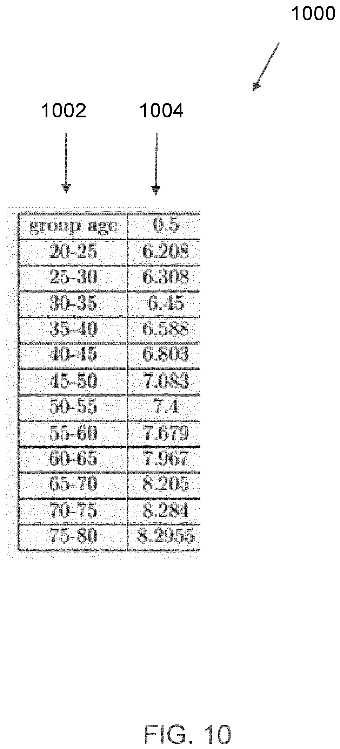

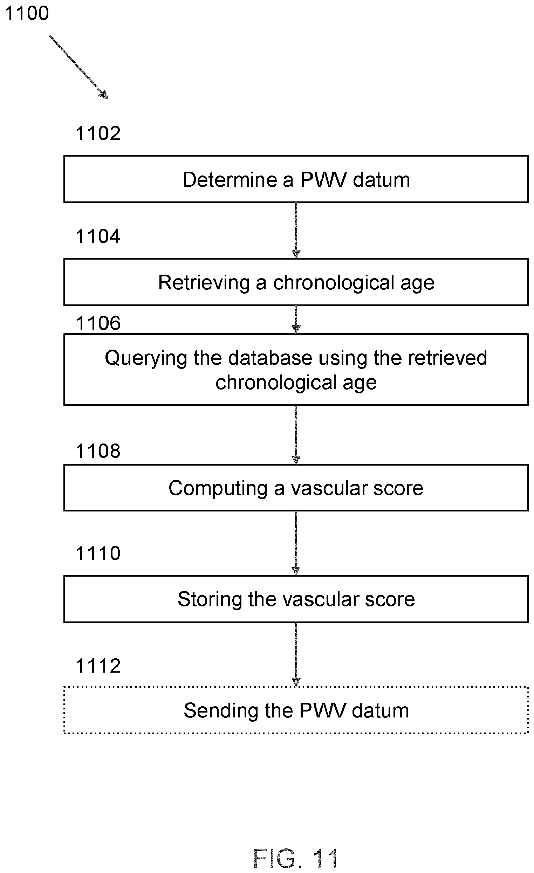

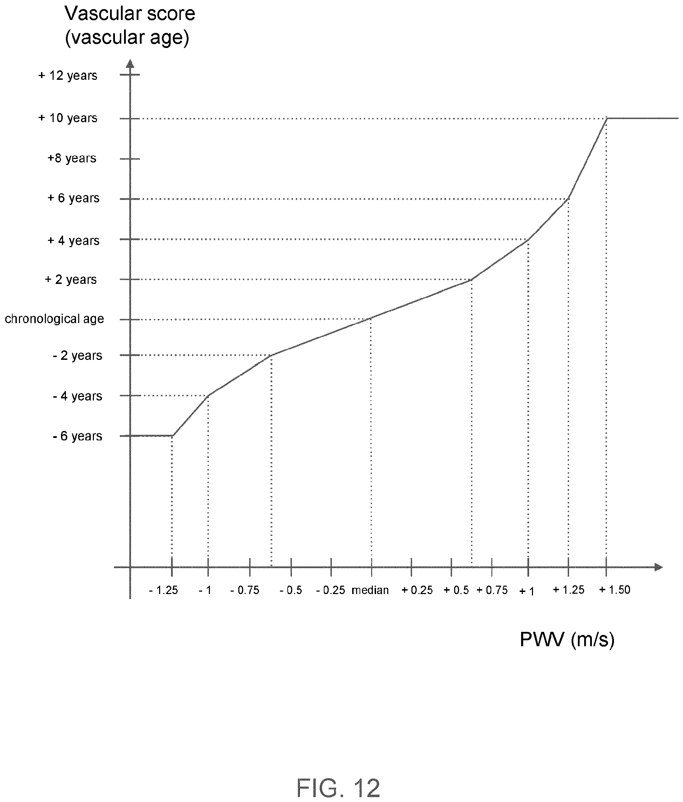

. The above and other objects and advantages of the disclosure will be apparent upon consideration of the following detailed description, taken in conjunction with the accompanying drawings, in which like reference characters refer to like parts throughout, and in which:
. FIG. 1 illustrates a schematic representation of a method, according to an aspect of the disclosure;
. FIG. 2 illustrates a hardware configuration of a system, according to another aspect of the disclosure;
. FIG. 3 illustrates a body of a user standing on a weighing scale, according to an aspect of the disclosure;
. FIG. 4 illustrates a flowchart representing a method to generate the database, according to an aspect of the disclosure;
. FIG. 5 illustrates a database, according to an aspect of the disclosure;
. FIG. 6 illustrates a flowchart representing a method to compute a vascular score according to an aspect of the disclosure;
. FIG. 7 illustrates a predetermined function to compute a vascular age as a function of the retrieved quantile, according to an aspect of the disclosure;
. FIG. 8 illustrates the distribution of PWV-related data for age group [30; 35[; as obtained on a fleet of user measuring devices;
. FIG. 9 illustrates another predetermined function to compute a vascular age as a function of the retrieved quantile, according to an aspect of the disclosure ;
. FIG. 10 illustrates a database, according to another aspect of the disclosure;
. FIG. 11 illustrates a flowchart representing a method to compute a vascular score according to another aspect of the disclosure;
. FIG. 12 illustrates a predetermined function to compute a vascular age as a function of the retrieved PWV-related datum, according to another aspect of the disclosure.

### DETAILED DESCRIPTION

. A process and device or system according to some embodiments of the disclosure compute and generate for display to a user a cardiovascular health related score (now referred to as a "vascular score") associated with a human body, for example to display to the user his or her vascular score and that of his or her next of a kin). The vascular score may be expressed as a number, the unit of which is an age. The vascular score may alternatively be expressed as a series of alphanumeric characters, such as words (*"optimal", "not optimal", "younger", "older", "+"*, "*-"*, etc.). The vascular score is stored and generated to be displayed so that the user may read it and easily have access to information about his or her cardiovascular health.

### Overview

. FIG. 1 illustrates an overview of a process according to some embodiments of the disclosure. In particular, the whole process contains two parts, which can be carried out independently: one part comprises generating a database DB and another part comprises generating a vascular score using that database DB. The database DB may be generated by a server 101, by means of a fleet 102 of measuring devices 103 (such as a weighting scales) and user devices 104, such as smartphones connected to the measuring device 103. Each measuring device 103 may be paired with at least one user device 104 and reciprocally. The fleet 102 may communicate with the server 101 using a communication network 105 (including a local network such as Bluetooth, Wi-Fi and/or a global network such as the internet). The double arrows indicate that a two-way communication is enabled.

. Fleet 102 is used to generate a database of physiological data. The physiologic datum is obtained by measuring a biomarker on a user. In an implementation, the physiological datum is a metric representative of a time characteristic and a distance of a pulse wave. In particular, such metric may either be a PWV value or a PWV-related value. By PWV-related value, it is meant that the metric is homogenous of a speed and concerns a pulse wave running through a portion of the vascular system of the user. With the present disclosure, whether that metric is a PWV of medical grade or not is mitigated, as it will be observed that the computed vascular score is not affected by any offset or discrepancies between medical grade PWV values and PWV-related values which may present a bias or an offset. In particular, if the PWV-related datum show discrepancies compared to a medical PWV based on an age group, the present disclosure proposes a solution that overcomes this issue. For example, if the PWV-related data obtained using the fleet 102 of measuring devices 103 are consistently below PWV values of medical grade for certain age group (such as the older the age group, the wider the discrepancy), those differences will be mitigated by means of the process of the present disclosure.

. In one implementation, the measuring devices 103 of the fleet 102 are all identical, that is to say a same model from a same manufacturer is used. At least, the same modules (sensors and algorithms) to obtain the metrics need to be identical between all the measuring devices 103 of the fleet 102. This ensures a high level of repeatability and consistency of the measures. Tests may be run between different models of measuring devices to ensure that the PWV-related data are consistent; if they are, then they can be used to populate a same database DB.

. How the measuring device may obtain the PWV-related datum will be disclosed in more details below.

. In a step E1, server 101 receives from fleet 102, through the communication network 105, a plurality of PWV-related data from a sample of users. The sample may contain more than a thousand users. The results shown in the present disclosure involve a sample of more than a hundred thousand users, from twenty to eighty years of age. The bigger the size of the sample, the more accurate the method of the disclosure is. Each of those PWV-related data is linked with at least one other user-specific data: his or her chronological age. The chronological age may be input by a user on a user device 104 (which may be paired with the measuring device), for instance during the set-up of the measuring device 104. Therefore, each measuring device 103 or each user device 104 sends to the server 101 a pair of data, including the PWV-related datum and the chronological age datum of the user associated with the PWV-related datum. In a step E2, the server 101 gathers all the pairs PWV-related data / chronological data and generates a database DB comprising, for each age group (chronological age or range of chronological ages), a repartition of the values of the PWV-related data (repartition by quantile). For instance, for different age ranges, such as [30;35[ or [35; 40[, the database may include the PWV-related datum of each decile or each percentile of the sample (or any other quantile). A measuring device may be a weighing scale, that is widespread amongst different populations around the world, so that the database is heavily populated with thousands of PWV-associated values. The amount of data further allows to split the sample of users into refined quantiles, such as percentiles instead of deciles, while keeping a high level of precision. More details about the database will be given later. In an implementation, at step E3, the database DB, which is light in terms of data (usually a few kilo-octets), and therefore wieldy, may be distributed to each measuring device 103 of the fleet and in particular on measuring device 106 which a user is about to use in order to obtain some information about his or her cardiovascular health. In another implementation, at step E3, the database is sent to the user device 104 of the fleet and in particular on user device 108 of the user who is about to obtain some information about his or her cardiovascular health. Alternatively, the database DB is stored and kept in the server 101 only.

. In a step F1, a measuring device 106, which is similar to those of the fleet 102, determines a PWV-related datum of a user that wishes to obtain a vascular score. This PWV-related datum is also linked with at least one other user-specific datum, his or her chronological age, to create a pair of data. The chronological age may have been input beforehand by the user via a user device 108 which may be paired with the measuring device 107. In a step F2, measuring device 107, using the PWV-related data, the database DB (received following step E3) and a predetermined law, computes a vascular score based on the chronological age of the user and the quantile of the database BD associated with the PWV-related datum of step F1 (i.e. the intervals of quantiles in which the PWV-related value falls). The vascular score gives the user an indication about his or her cardiovascular age. More details about the vascular score will be explained later. In particular, no regression (i.e. interpolation of data) may be involved to compute the vascular score). In a step F3, the measuring device 107 generates for display the vascular score on a screen and/or, in a step F4, sends the vascular score to a user device 108 which displays it. Alternatively, the measuring device 106 may send the PWV-related datum to the user device 108 which computes the score as explained. The user device 108 then generates for display said vascular score.

. Alternatively, when the database BD is not distributed but kept on the server, the PWV-related measure is sent to the server 101 by the measuring device 106 through the communication network 105, such that the server 101 may compute the score.

. To further enrich the database, in a step F5, server 101 updates the database DB with the pair PWV-related datum (obtained at step F1) / chronological age of the user. To that end, if not already done, the pair of data is sent by the measuring device 106 or the user device 108 to the server 101. The more pairs of data the database contains, the more refined and precise the database.

### Architecture

. FIG. 2 illustrates a measuring device 200 (which may be the measuring devices 103 and 106), a user device 220 (which may be the user device 104 and 108), a server 230 (which may be the server 301), linked together through the communication network 105. The communication network may include wireless communications (BlueTooth, Wi-Fi, etc.), local networks, global networks, cellular network, Internet, etc.

. The measuring device 200 comprises control circuitry 202, a user interface 204 (for example a screen), a wireless module 206, a weight sensor 208 and an impedance sensor 210. Control circuitry 202 includes processing circuitry 212 and storage 214. Control circuitry 202 may be based on any suitable processing circuitry (one or more microprocessors, microcontrollers, etc.). Control circuitry 202 is connected to the user interface 204 to receive therefrom or display information thereon. In an embodiment, the user interface 202 is a display. Control circuitry 202 is also connected to the wireless module 206 to send data to, and receive data from, the communication network 105. The weight sensor 208 is configured to measure the weight of a user standing on the measuring device 107 and to measure a BCG. The weight sensor 208 may include four load cells for instance. The impedance sensor 210, which includes a set of electrodes, is configured to perform an IPG, and, if needed, to perform a bioelectrical impedance analysis (BIA) to determine a composition of the user body (fat, water, bone, muscle, etc.). Documents US2016317043 and US2017065185 (assignee: Withings), which are herein incorporated by reference, describe an implementation of the measuring device 200. Amongst devices that are or have been on sale, such a measuring device is Withings Body Cardio.

. The user device 220, which may be a smartphone, a tablet or a computer, comprises control circuitry 222, a user interface 224 (for example a touch screen), and a wireless module 226. Control circuitry 222 includes processing circuitry and storage (not illustrated). Control circuitry 222 is connected to the wireless module 226 to send data to, and receive data from, the communication network 105.

. The server 230 also comprises control circuitry 232 and an I/O interface 234 to receive data from, and send data to, the communication network 105. Server 230 is configured to process and store data. Control circuitry 232 includes processing circuitry 236 and storage 238 to handle and store a database populated from data from the measuring devices 200 and the user device 220.

. In one embodiment, a user device 220 may be paired with a measuring device 200, such that any information measured by the measuring device is automatically sent to the user device, upon synchronization, and any information input on the user device may be sent to the measuring device.

. In one embodiment, the measuring device 200 may communicate directly with the user device 220 via Bluetooth or Bluetooth Low Emission (with the wireless module 206). In this embodiment, the measuring device 200 may indirectly communicate with the server 230 through the user device 220. In an alternative embodiment, the measuring device 200 may communicate indirectly with the user device 220 via a WiFi network.

. In another embodiment, the measuring device is a blood pressure monitor which may integrates a sound sensor, such as that disclosed in US2020214577 (assignee: Withings), whose content is herein incorporated by reference. Amongst devices that are or have been on sale, such a measuring device is Withings BPM Core.

. More generally, any measuring device that is able to determine a PWV-related datum may be used. However, the measuring device is advantageously part of the "consumer electronics" realm, that is to say a piece of electronic equipment intended for everyday use, typically in private home. This means that the measuring device is a widely spread device, so that thousands of data can be easily gathered. In an implementation, the measuring device is configured to generate a PWV-related datum using at least one measuring session that lasts less than 30s, even less than 15s each. This ensures that the user will regularly use the measuring device to determine a PWV-related datum.

### How the measuring device determines the PWV-related datum

. The PWV-related datum may be determined according to the methods and techniques disclosed in previously cited document US2017065185. In particular, said PWV-related datum M is determined as M=f(L/DT) where f is a function, L is a characteristic path length of the user body and DT is a characteristic time, where DT is measured by the measuring device. More generally, characteristic path length L (hereby referred to as "the length L" for concision reasons) is a length associated with a path P (a path of arteries) along which the pulse runs. FIG. 3 illustrates a configuration 300 with a body of a user U standing on a measuring device 200 according to an implementation.

. Depending on the method to obtain the characteristic time DT, length L may vary accordingly. Length L may stem from the heart to the waist (between the legs, at the femoral artery), from the heart to the foot, from the heart to the arm, from the heart to the wrist, from the heart to the hand, from the heart to the neck or any combination thereof (from the neck to the foot or the waist), etc. In FIG. 3, path P is defined between the heart 302 of the user U and the feet 304, 306 (or at least one foot). Path P thus includes the aorta 308, the femoral artery 310 and the tibial artery 312.

. In any case, to ensure that the computed vascular score is related to the cardiovascular health of the user, the length L is to be related to a path that includes a portion of the aorta. The path L may therefore include a portion of the artery between the heart and the common carotid artery and/or between the heart and the femoral artery. The most common PWV method found in the literature is the carotid to femoral length, as used in the cfPWV (carotid to femoral PWV). However, when the measuring device 200 is a weighting scale as illustrated on FIG. 3, a cfPWV measure is not achievable, as path P extends between the heart 302 and the foot 306, 308. Length L is related to the actual length of the path P in the arteries but is often a simplified value, such as the direct distance between the extremities of the path P.

. Length L may be inputted by the user on the user device 220 or may be calculated as a function of the height of the user (which may also be combined with the gender of the user) which may have been inputted by the user on the user device 220. Any other method to determine length L may be used.

. Characteristic time DT (hereby referred as "time DT") is the time the pulse takes to run along the path P. Time DT is related to a pulse transit time (PTT).

. DT is generally computed as a time difference between two instants T1, T2, which identify (directly or indirectly) the time at which the pulse is at the beginning of the path P and the time at which the pulse reaches the end of the path P. In an implementation, instant T1 may be the opening of the aortic valve of the heart. Instant T1 may be determined by means of a ballistocardiogram (BCG) or a phonocardiogram. For example, the weight sensors 208 of the measuring device 200 are configured to perform a BCG. In an implementation, instant T1 may be determined by means of at least an impedance plethysmograph (IPG) or a photoplethysmogram (PPG). In an implementation, instant T2 may be obtained by means of an IPG or a PPG. For example, the impedance sensor 210 of the measuring device 200 are configured to perform an IPG. In particular, the difference T2-T1 may be obtained by means of two IPGs and/or PPGs at the extremities of the path P. Other techniques are possible, including techniques not yet developed.

. The function f is a parametrization which may depend on the age and gender of the user, and also may depend on, albeit in a lesser extent, a blood pressure type (normal, hypertensed, ...) and the height/weight of the user and optionally also the blood pressure type. In an implementation, f is the identity function and the PWV-related datum M is M=L/DT. Although this datum M might not be a PWV datum *per se* under a medical definition, datum M still contains some information about the cardiovascular health of the user and, thanks to the approach of the present disclosure, any difference with a medical PWV measure (such as a cfPWV) is compensated by the statistical approach. In addition, using the identity function f (therefore M=L/DT) simplifies all the processing needs in terms of hardware and software.

. The PWV-related datum may be determined by other devices, as long as the device is able to determine time DT (length L is usually obtained by other means, as explained above: input of the user on the user device for instance). Finger devices, such as devices pinching the tip of a finger, may be used; improved blood pressure monitor may be used as well.

. The measuring device therefore enables to obtain a PWV-related datum M, which has the unit of a speed (m/s for instance). As explained above, datum M may not be a medical PWV datum and is therefore referred to as a PWV-related datum. By PWV-related, it is meant that the PWV-related datum contains some information about the velocity of the pulse wave running through a portion of the aorta and, thus, some information about the cardiovascular health of the user. The PWV-related datum may be sent by the measuring device to the server or the user device (such as a smartphone) via the communication network. Alternatively, when the measuring device does not compute the PVW-related datum but only the characteristic time DT, the measuring device sends the datum of the time DT to the user device or the server which then computes the PWV-related datum.

. To be usable as a meaningful score linked to a vascular age, the PWV-related datum preferably involves a path P going through at least a portion of the aorta. Other types of measures are technically implementable but the relation with a vascular age is more distant and therefore less relevant to characterize the vascular age of a subject. However, the present disclosure also applies to determine an information about the vascular health of any portion of the human body.

. As explained before, the present disclosure includes two different parts. A first part relates to the database and a second part relates to the use of that database.

. In view of the rest of the disclosure, it is assumed that the measuring device, combined with the user device or the server, is able to determine a PWV-related datum.

### Generation of the database

. In relation to the flowchart of FIG. 4, a process 400 to generate a database DB will be described. The database BD is illustrated on FIG. 4. Process 400 may be carried out by control circuitry 232 of the server 230.

. At step 402, control circuitry 232 receives, from each measuring devise 103 of fleet 102, a plurality of PWV-related data. The PWV-related datum may be received along with a chronological age datum of the user to which the PWV-related datum is associated. This ensures confidentiality and anonymity of the data, as the server needs not to store any identifier about the measuring device or the user. Alternatively, to avoid sending the chronological age datum with the PWV-related datum, control circuitry 232 may receive a PWV-related datum along with a user identifier. Server 230 then associates the PWV-related datum with a chronological age datum stored, that is itself associated with the user identifier. In both cases, the server retrieves a plurality of pairs of data: the PWV-related datum and the chronological age datum. In an implementation, at least a thousand pairs of data are retrieved by control circuitry 232. In an implementation, at least then thousand pairs are retrieved. In an implementation, at least one hundred thousand pairs are retrieved. Alternatively, control circuity 232 receives from each measuring device 103 of fleet 102 a time DT and retrieves a length L to compute the PWV-related datum at the server level.

. To generate a PWV-related datum, a plurality of measures may be carried out on a same user, so that the PWV-related datum is an average value or a median value of PWV-related measures. In an implementation, five measures are required to generate the PWV-related datum. For example, five measures within a month are needed to generate a PWV-related datum.

. The database DB may include one PWV-related datum per user. Alternatively, the database may include several PWV-related data per user, for example spread over time (one PWV-related datum every year for a user). To maintain the same weighing in the database, it is preferable to have the same number of PWV-related data for each user.

. As mentioned above, the PWV-related data need to be consistent in order to be part of a same database. To that end, the user measuring devices 103 of fleet 102 are either all identical (same model or same specifications) or the relevant modules to determine the PWV-related datum (weighting sensor, impedance sensor, algorithms, etc.) are identical (same model or same specifications). If not, tests can be run to ensure that the PWV-related data are homogenous for each age group between the two models or types of measuring devices.

. At step 404, control circuitry 232 computes the database DB using the plurality of retrieved pairs of data. Database DB 500, as illustrated in FIG. 5, contains, for a plurality of chronological age groups 502 (referred to as the age groups), a repartition of the PWV-related data, that is to say, for a plurality of quantiles 504 (for each age group), a PWV-related value associated with a quantile. A quantile is a cut point dividing the sample into intervals. The quantiles may be uniform (for examples deciles or percentiles) or have different sizes.

. For example, for age group [20-25[, 5.35 m/s is the PWV-related value that delimits the first 10% of the PWV-related data and 6.681 m/s is the PWV-related value that delimits the first 75% of the PWV-related data of the sample (both of users from the sample, the chronological age of whom is between [20; 25[). In other words, for each age range, the PWV-related data are ranked from the lowest to the highest value and the value of the PWV-related data that indicates the first X % of the total number of PWV-related data is the value associated with the X^{th} quantile. All the PWV-related data that are below said value associated with the X^{th} quantile ("QX") are part of that quantile. For example, the value of the PWV-related data for the first 10% of the sample defines the PWV-related value associated with the quantile 0.1 ("Q10").

. In an implementation, an age group is a year, so that the database DB contains an entry for each chronological age. In another implementation, an age group is an age range defined by intervals of Z years, wherein Z is comprised between 2 and 10 (for example 5, as illustrated on FIG. 5). The age range may start at any age, such as 20 years old, and finish at any age, such as 80 years old. Other units than years may be used (years and months, months, or even seconds and any data may be converted in another unit).

. The quantiles may be uniformly spread, so that the sample is uniformly divided. The quantiles may thus be deciles (10^{th}: Q0, Q10, Q20, ...., Q90, Q100)) or percentiles (100^{th}: Q0, Q1, Q2..., Q49, Q50, Q51, ..., Q99, Q100), but other divisions are possible, such as 20^{th} (Q0, Q20, Q40, .... Q80, Q100) or quartile 25^{th} (Q0, Q25, ...Q75, Q100). In an implementation, the quantiles are not uniform, so that for example a first quantile Q10 encompasses the first 10% of the sample, the second quantile Q25 encompasses 25%, the third quantiles Q50 50%, the fourth quantile Q75 75%, the fifth quantile Q90 90% and the sixth quantile 100%. As CVD risks are higher with a high PWV-related data, it may be relevant to refine more precisely the database for quantiles above Q50. Therefore, there might be fewer quantiles below Q50 than quantiles above Q50. Any other divisions are possible. The smaller the quantile, the more precise the database. In particular, the bigger the sample, the smaller the quantiles may be while keeping a high level of precision (as they would still be many PWV-related data in each quantiles).

. Database DB 500 of FIG. 5 may be a full database DB according to an implementation or a partial view of a fuller database BD (for example with percentiles).

. As presented on FIG. 5, the database DB is presented in a cumulative way. The quantile into which a PWV-related datum falls is actually an interval. When the quantiles are centiles, it would be possible to indicate that the PWV-related datum falls into an interval defined by two consecutive centiles, for which the PWV-related datum is higher than the PWV-related value of the lower centile and the PWV-related datum is lower than the PWV-related value of the higher centile. Therefore, when querying the database DB, those two centiles are identified, using the pair of data as input (chronological age datum and PWV-related datum).

. Database DB only contains a set of data with a limited number of figures, as database DB does not store any of the PWV-related data (except those associated with each quantile). In an example, database DB may contain 100 centiles and around 15 age groups, thus around 1500 figures. In another implementation, database DB may contain 10 deciles and around 12 age groups, thus around 120 figures. In any case, database DB is light and can easily be sent through the communication interface, whether cellular communications, Wi-Fi or Bluetooth are involved.

. At step 406, control circuitry 232 stores the database DB in storage 238.

. At step 408, control circuitry 232 sends the database DB to the measuring device 200. For instance, all the measuring devices of the fleet 102 receives the database BD. In an implementation, control circuitry 324 sends the database DB to the measuring device 200 via the user device 220. Alternatively, control circuitry 232 sends the database BD to the use device 220. Alternatively, database BD is not sent and step 408 is not carried out.

. The database is advantageously dynamic, that is to say it is regularly updated with new PWV-related data. For instance, each time the measuring device 220 determines a PWV-related datum of the user or every time a few measures are carried out by the measuring device 200, an update may be sent to the server 230.

. Once a database DB is ready to use, any measuring device having access to the database (either directly or indirectly) is able to compute a vascular age for a user.

### Computation of a vascular score, such as a vascular age

. In relation to the flowchart of FIG. 6, a process 600 to compute a vascular score will be described. Process 600 may be carried out by control circuitry 202 of the measuring device 200. Other control circuitry may be involved as well.

. At step 602, control circuitry 202 retrieves a PWV-related datum of a user. How the PWV-related datum is obtained was detailed above. Retrieving may include determining, in particular when control circuitry is that of the measuring device. If the control circuity is not that of the measuring device, retrieving may comprise receiving. Step 602 may involve a plurality of measures, so that the PWV-related datum is an average value or a median value of PWV-related measures. In an implementation, five measures are required to generate the PWV-related datum.

. At step 604, control circuitry 204 retrieves a chronological age datum of the user. The user is the same user as the one for which the PWV-related datum was obtained. As already detailed, the chronological age datum may already be stored in the measuring device 200, such that control circuitry 204 has immediate access to it. Alternatively, the chronological age datum may be received from the user device 220 that is paired with the measuring device 200. The PWV-related datum and the chronological age datum are associated to form a pair of data.

. At step 606, control circuitry 204 retrieves from the database DB (which may be stored by control circuitry 204) the value of the quantile into which the PWV-related datum falls, for the age group into which the age datum falls. In other words, the database DB is queried with the chronological age datum and PWV-related datum to return a value of a quantile. Step 606 does not require heavy processing circuitry and may be perform quickly on the measuring device 200. More precisely, the returned quantile may be that for which the PWV-related value associated with the quantile is the closest inferior or the closest superior to the retrieved PWV-related datum.

. At step 608, control circuitry 204 computes a vascular score using the returned quantile, the chronological age datum and a predetermined law (or predetermined function). The vascular score is therefore directly dependent on the actual chronological age of the user. This allows to give a vascular score that is relevant to the user. Indeed, as may be observed in database DB 500 of FIG. 5, there is an important overlap between the intervals defined by the lowest and highest values of the PWV-related data for each age group. For example, a PWV-related datum of 7.35 m/s may belong to a user aged [20; 25[ (between quantiles 0.75 and 0.9) or a user aged [75; 80[ (between quantiles 0.1 and 0.25). As such, the PWV-related datum may not suffice to provide a relevant vascular score for the user.

. Other techniques might query the database DB with the PWV-related datum only, in order to return different age groups with their associated quantiles. An algorithm using the returned age groups with their associated quantiles may be used to determine a vascular score. However, a selection or a weighting needs to be carried out and that would further require information about the user, which is precisely an issue that the present disclosure proposes to overcome. The vascular ages of the literature require more than three data about the users, while the present disclosure may only use the chronological age and the PWV-related data (the PWV-related datum involving at least two different data: length L and time DT).

. The predetermined law (or function) may return a score that has the unit of a chronological age (so-called "vascular age"). The precision of the vascular age may have one digit after the coma (such as 45.2 years old or 23.9 years old, although the units could be years and months, or months ; in any case, unit conversion remains possible). Alternatively, the predetermined function may return an alphanumeric chain (such as words or symbols), or may convert the vascular age into an alphanumeric chain.

. In an implementation, the predetermined law F is a linear function whose variables are the retrieved quantile and the chronological age: F(Q, Age) = Age + (αQ+β) where α, β are parameters and the retrieve quantile Q ranges from 0 to 1. The value of the vascular score directly depends on parameters α, β. Typically, for Q50, which is Q=0.5, (when using percentiles: either PWV-related datum falling between Q49 and Q50 or PWV-related datum falling between Q50 and Q51), it may be desired that the vascular age corresponds to the chronological age. Then, it may be desired that the same maximum difference of age Ω (in years) is reached at Q0 and/or Q100, then both parameters are settled: β = - Ω and α = -2 β. For example, if it is decided that Ω = 10 years, then F(Q, Age) = Age + 20Q- 10.

. For all implementations, Ω is typically comprised between 5 and 20 years, even 2 and 40 years.

. In an implementation, the predetermined law F is a parametric piecewise function whose variables are the retrieved quantile and the chronological age and whose parameters vary based on the retrieved quantile. Under a mathematical formulation, predetermined law F may be as follows: function F(Q, Age) = F_{Q}(Q, Age), where the parameters of F_{Q} depends on Q. More precisely, the parameters of F_{Q} depends on an interval of quantiles into which Q falls, as explained above.

. In an implementation, predetermined piecewise law F is a function which has at least two different expressions for two different intervals of the retrieved quantile Q: for Q_{A} ≤ Q < Q_{B}, F may be defined as F_{AB} (Q, Age), and for Q_{C} ≤ Q < Q_{D}, F may be as F_{CD}(Q, Age), where Q_{B} ≤ Q_{C}. Symbol "≤" and "<" may be defined differently, as it is only a matter of design. In an implementation of that function F, at least one boundary of an interval of the retrieved quantile is comprised between 0.1 and 0.4 (both included) and/or at least a boundary of another interval of the retrieved quantile is comprised between 0.6 and 0.9 (both included). For example, as it will be detailed later, the intervals of the pieces may be [0; 0.25[, [0.25; 0.5[, [0.5; 0.75[, [0.75; 1[ or [0; 0.10[, [0.10; 0.25[, [0.25; 0.75[, [0.75; 0.9[, [0.90;1[.

. For instance, in one implementation, predetermined law F is a 5-piecewise function, with for interval Q_{A} ≤ Q < Q_{B} F, = F_{AB}(Q, Age); for Q_{C} ≤ Q < Q_{D}, F_{CD}(Q, Age); for interval Q_{E} ≤ Q < Q_{F}, F_{EF}(Q, Age); for interval Q_{G} ≤ Q < Q_{H}, F_{GH}(Q, Age); for interval Qₗ ≤ Q < Q_{J}, F_{IJ}(Q, Age). To cover the whole distribution of quantiles, we may have Q_{A} = 0, Q_{B} = Qc, Q_{D} = Q_{E}, Q_{F} = Q_{G}, Q_{H} = Q_{I}, Q_{J} = 1. Q_{A}, Q_{B}, Q_{C}, Q_{D}, Q_{E}, Q_{F}, Q_{G}, Q_{H}, Q_{I}, Q_{J} may have the values at the previous paragraph.

. In an implementation, the predetermined parametric piecewise function F is a linear function: F(x) = αx+β, where α, β are parameters. Each piece of the piecewise function F may be a linear function. Written differently, F_{XY}(Q, Age) = Age + (α_{XY}Q+β_{XY}), where (α_{XY}, β_{XY}) are parameters depends on the interval defined by quantiles Q_{X} and Q_{Y}.

. In an implementation, the piecewise law is continuous. This means that, using the generic example of above when Q_{B} = Qc, F_{AB}(Q_{B}, Age) = F_{CD} (Q_{C}, Age). FIG. 7 illustrates the shape of such a continuous piecewise function.

. The parameters of the law F directly determine the value of the vascular score. They therefore need to be chosen in order not to mitigate the influence of (Q, Age). Depending on the vascular score, the units of the parameters may change. In one implementation, the vascular score is a vascular age and therefore the units of the parameters are in yrs.s/m (year second per meter) or in years. In one implementation, to keep a score within a meaningful interval, the parameters are chosen so that the vascular age is not beyond the chronological age plus or minus Ω years, where Ω is comprised between 5 and 20 years (for instance 10 years). Using the mathematical form expressed above, this translates into parameters α_{XY}, β_{XY} chosen so that F_{XY}(Q, Age) - Age = α_{XY}Q+β_{XY} is comprised between [-Ω years ; + Ω years] in any case.

. For the 5-piecewise function described above, the parameters may be chosen so that the predetermined functions is made of 5 subfunctions:
vascular age = chronological age - 10 years for PWV-related datum falling next to Q0 (when using percentiles: between Q0 and Q1),
vascular age = chronological age - 6 years for PWV-related datum falling next to Q10 (when using percentiles: between Q10 and Q11),
vascular age = chronological age - 2 years for PWV-related datum falling next to Q25 (when using percentiles: between Q25 and Q26),
vascular age = chronological age for PWV-related datum falling next to Q50 (when using percentiles: between Q50 and Q51),
vascular age = chronological age + 2 years for PWV-related datum falling next to Q75 (when using percentiles: between Q75 and Q76),
vascular age = chronological age + 6 years for PWV-related datum falling next to Q75 (when using percentiles: between Q75 and Q76),
vascular age = chronological age + 10 years for PWV-related datum falling next to Q100 (when using percentiles: being Q100).
In this predetermined function, the shape linking each of two consecutive points is straight. Under a proper mathematical form:
   for 0≤ Q < 0.1, F_{AB}(Q, Age) = Age + (Q-0.1) x 40 - 6
   for 0.1 ≤ Q < 0.25, F_{CD}(Q, Age) = Age + (Q-0.25) x (4/0.15) - 2
   for 0.25 ≤ Q < 0.75, F_{EF}(Q, Age) = Age + (Q-0.50) x 8 + 0
   for 0.75 ≤ Q < 0.90, F_{GH}Q, Age) = Age + (Q-75) x (4/0.15) + 2
   for 0.90≤ Q < 1, F_{IJ}(Q, Age) = Age + (Q-0.9) x 40 + 6

The values of - 10 years, - 6 years, - 2 years, + 2 years, + 6 years, + 10 years may be changed.

. In one implementation, as illustrated with the example of FIG. 7, the vascular score (here a vascular age) is symmetrical around Q50 (Q=0.50).

. FIG. 8 illustrates the distribution of PWV-related data for the age group of [30-35[: it is observed that most of the values are concentrated and fewer values are spread out. To account for this non-uniform distribution, the predetermined law F may provide a score whose variation depends on how far from Q50 the returned quantile is. For the linear piecewise function, this means that the slope of each linear subfunction constituting the predetermined function may vary. The further from Q50 the interval of definition associated with the subfunction, the steeper the slope. Again, this has been illustrated with the example of FIG. 7, where the slope is the steepest for intervals [Q0; Q10[ and [Q90; Q100[ which are the farthest from Q50, and the slope is less steep for the intervals [Q10; Q25[ and [Q75; Q90[ which are closer to Q50, and the slope is even less steep for the interval [Q25; Q50[, as it includes Q50. More concretely and as an example only, the difference of vascular scores between two users whose PWV-related values belong to Q45-Q50 is smaller than the difference of vascular scores between two users whose PWV-related values belong to Q90-Q95.

. However, as may still be observed in FIG. 8, the distribution of PWV-related data is not entirely symmetrical around the apex. To account for this lack of symmetry, the predetermined law F may provide a score whose variation is not symmetrical around Q50. More particularly, the steepest slope above Q50 is steeper than the steepest slope below Q50. FIG. 9 illustrates a 5^{th} piece linear function where, at Q=0, the difference between the chronological age and the vascular age is - 10 years and, at Q=100, the difference between the chronological age and the vascular age is + 12 years. The parameters defining each F_{XY}(Q, Age) may be adapted so that the variation of the score (i.e. the derivative or the slope) is the highest at the farthest above Q50. This may be combined with the implementation described at the previous paragraph (which corresponds to the function of FIG. 8).

. Still to account for the lack of symmetry observed in FIG. 8, the intervals of definition of the pieces of the predetermined piecewise function F may (complementarily or alternatively) be asymmetrical around Q50. For instance, written as Q_{X} ≤ Q < Q_{Y} F = F_{XY}(Q, Age), intervals [Qₓ; Q_{y}[ may have different sizes. As discussed previously, the vascular score is particularly relevant for people with premature vascular aging. Therefore, below Q50, the predetermined piecewise function F may be defined differently on only a few (for instance one or two) intervals [Qₓ; Q_{y}[, such as [Q0; Q10[ and [Q10; Q50[. In an extreme implementation, an interval [Qₓ; Q_{y}[ is [Q0; Q_{Y}[, where Q_{Y} is equal or above Q50. Conversely, above Q50, a higher division (or higher granulometry) may be used with more intervals [Qₓ; Q_{y}[ (for instance three, four or five), such as [Q50; Q75[, [Q75; Q90[, [Q90; Q95 [, [Q95; Q100[.

. The predetermined law F described above do not depend on the retrieved chronological age. However, as noted earlier, vascular age may be more relevant for older people. To account for that principle, the parameters of the predetermined function F may depend on the retrieved chronological age. For example, the predetermined function F of FIG. 7 and 9 may have a different shape based on the retrieved chronological age. In an implementation, the older the retrieved chronological age, the higher the maximum age difference Ω: for age group [20; 25[, Ω may be comprised between 2 and 6 years and for age group [75; 80[, Ω may be comprised between 8 and 12 years. In an implementation, the predetermined function F is a parametric piecewise function whose variables are the retrieved quantile and the chronological age and where the parameters of the function vary based on the retrieved quantile and the retrieved chronological age.

. In an implementation, predetermined piecewise law F is a function which has at least two different expressions for two different intervals of the retrieved quantile Q and for two different age groups of the retrieved chronological age datum: for Q_{A} ≤ Q < Q_{B} and Age_{A} ≤ Age < Age_{B}, F may be defined as F_{AB}^{AgeAB} (Q, Age), and for Q_{C} ≤ Q < Q_{D} and Age_{C} ≤ Age < Age_{D} F may be as F_{CD}^{AgeCD}(Q, Age), where Q_{B} ≤ Qcand Age_{B} ≤ Age_{C}. More precisely, for Q_{A} ≤ Q < Q_{B} and Age_{A} ≤ Age < Age_{B}, F may be defined as F_{AB}^{AgeAB} (Q, Age), and for Q_{A} ≤ Q < Q_{B} and Age_{C} ≤ Age < Age_{D} F may be as F_{AB}^{AgeCD} (Q, Age), where Age_{B} ≤ Age_{C}

. Other values of the parameters may be chosen. However, a goal of the vascular score is to provide an easy-to-understand metrics about the user's body and a valuable metric. In other words, the metric shall not lead the user to believe something that is somewhat meaningless: for instance, telling someone that his or her vascular age is 40 years younger than their chronological age may be deceptive, as some medical vascular age (as those computed in specialized institutions) takes into account more variables into the calculation.

. As visible from the description, the proposed technique to compute a vascular score may involve no regression or interpolation of data.

. At step 610, control circuitry 204 stores the vascular score. At step 612, control circuitry 204 may generate for display the vascular score or any indication directly related to the vascular score. For instance, when the vascular score is a vascular age, control circuitry 204 may generate for display the vascular score itself or an indication comprising a text such as "much older", "older", "same age", "younger", "much younger", "optimal" or an alphanumeric character "++", "+", "=", "-", "-" based on a comparison between the chronological age of the user and the vascular age.

. The indication related to the vascular score may be displayed on the user interface 204 of the measuring device 200 (the screen). The indication of vascular score may also (complementarily or alternatively) be sent to the user device 220 and displayed on a screen of the user device 220. In an implementation, the indication to be displayed on the measuring device 200 may be different from that to be displayed on the user device 220. For instance, a mere word may be displayed on the measuring device 220 ("optimal" for example) and the full vascular score ("56.7 years" for example) may be displayed on the user device 220.

. The vascular score may also be sent to the server 230, to gather vascular scores from the users and provide feedback.

. At a step 614, control circuitry 204 may send the retrieved PWV-related datum determined at step 602 to the server, to update the database. To that end, control circuitry 204 may send along the chronological age datum retrieved at step 604 (or the server may use an identifier sent with the PWV-related datum, as explained above). Step 614 may be carried out at different possible times of the process.

. Process 600 was disclosed as being carried out by the measuring device 200. However, process 600 may be performed by the user device 220 or by the server 230 itself (with the exception of the determination of the PWV-related datum that needs the measuring device 200). In those cases, the user device or the server receives the PWV-related datum and associates it with a chronological age (which is either sent along or associated by means of an identifier). The rest of the process is carried out in a similar manner.

### Alternative embodiment

. According to another embodiment, the present disclosure relates to a method in which the database only contains the PWV-related values for quantile 0.5 (or Q50), for each age group. FIG. 10 illustrates such as database DB 1000, which comprises, for a plurality of chronological age groups 1002, the median value 1004 of the PWV-related data (herein after referred to as the median). In other words, the database DB 1000 only store the median value (herein after referred to as the median) of the PWV-related data for each age group. The rest of the construction of the database is identical.

. The computation of the score will now be described in relation to the flowchart of FIG. 11, illustrating a process 1100 to compute a vascular score according to that other embodiment. Process 1100 may be carried out by control circuitry 202 of the measuring device 200. Other control circuitry may be involved as well.

. Steps 1102 and 1004 are similar to steps 602 and 604 and will not be described again. At step 1106, control circuitry 204 retrieves from the database DB (which may be stored by control circuitry 204) the median value of the PWV-related data for the age group corresponding to the retrieved chronological age. In other words, the database DB is queried with the chronological age datum to return the median value of PWV-related data for the age group into which the retrieved chronological age falls. Step 606 does not require heavy processing circuitry and may be perform quickly on the measuring device 200.

. At step 1108, control circuitry computes a vascular score using the returned median value of PWV-related data, the chronological age and a predetermined law. The predetermined law G compares the retrieved PWV-related datum with the median value and generates a vascular score. The predetermined law (of function) may return a score that has the unit of a chronological age. The predetermined law may take into account a difference between the retrieved PWV-related datum and the median value or a ratio between the retrieved PWV-related datum and the median value.

. For example, the predetermined law may be a function G as follows *G(PWV, Age) = Age* + *sign[PWV-median] × max[Ω; d × (PWV-median)],* where PWV is the retrieved PWV-related datum, Age is the retrieved chronological age, Ω is the maximum age difference (as described above) and ∂ is a parameter (the slope).

. In a simplified form, the predetermined law may be a function G as follows *G(PWV, Age) = Age* + *d × (PWV-median),* wherein ∂ is again a parameter. In that case there is theoretically no maximum for the computed vascular age but the PWV-related data has a limited value (the vascular age would be high for a few outliers but those people with such a high PWV-related datum could use see a high vascular age as meaningful).

. In other implementations, the predetermined law may be a piecewise function where the pieces are defined on intervals of the difference of *PWV-median* (as illustrated on FIG. 11). For example, there could be a subfunction defined for the *PWV* verifying *PWV ≤ median* and another subfunction for the PWV verifying *median < PWV.* Such piecewise function is preferably continuous. In more details, there could be more pieces based on the difference between *PWV* and *median* (i.e. the retrieved PWV-related datum and the returned median value) or a ratio between them. For example, a subfunction may have a set of parameters when *PWV*/*median* ≤ R_{A}, another set of parameters when R_{A} < *PWV*/*median* ≤ R_{B}, and another set of parameters when R_{B} < PWV/median and so on (more intervals may be defined), wherein R_{A}, R_{B}, etc. may be any value between 0.8 and 1.3 (for example). Those values R_{A}, R_{B} can be determined using the database DB 1000. Alternatively, as illustrated on FIG. 12, the intervals may be defined by differences *(PWV-median*) and not ratios. For each interval, a different slope may be determined, as illustrated on FIG. 12 again. As explained above in relation to process 600, the definition of the subfunction on each interval may follow some rules based on the differences between the central value (for process 600 above central value was quantile Q50 and here for process 1100 central is the median value of the PWV-related data). The farther away the *PWV* is from the *median,* the steeper the slope, as illustrated on FIG. 12 again. An exception may be made when a maximum Ω is defined (to flatten all the outliers), as illustrated on FIG. 12, for *PWV > media* + *1.5 m*/*s* and another maximum is defined *PWV < media* -1.25 *m*/*s.* The numbers given here are examples. The lack of symmetry may be accounted by having steeper slopes for *PWV > median* than for *PWV < median,* as illustrated on FIG. 12 again. For example the steepest slopes of the vascular score when *PWV > median* is steeper than the steepest slopes of the vascular score for *PWV < median.*

. All the sets of parameters of the predetermined function (or the subfunction for pieces of the predetermined function) may further depends on the retrieved chronological age. This allows to account for the changes in the spread of values of the PWV-related data between different group age. It is to be noted that these changes were automatically taken into account when using a database with many quantiles and when querying the database to return a precise quantile value using the retrieved PWV-related datum (and the chronological age). Here, changing the parameters of the predetermined function (or the pieces thereof) between age groups enable to alter the vascular age on that basis. This means that for a same difference *PWV-median*, the difference between the vascular age and the chronological age may change based on the age group of the user. This means that the shape of the predetermined function of FIG. 12 changes depending on the retrieved chronological datum (that is to say depending on the age group into which the retrieved chronological datum falls) associated with the retrieved PWV-related datum.

. As the vascular score includes a comparison with the median (either a subtraction or a ratio), the method also allows to at least partially compensate or offset any bias in the PWV-related data, albeit in a lesser extent than the embodiments of FIG. 4-9.

. The same considerations about control circuitry as described in relation to process 600 apply for the control circuitry which perform process 110.

### Other data that may be used to compute the vascular score

. To take into account specificities of certain populations, a geographical datum of the user may be used, such a country or a wider area (Europe, North America, etc.). When generating the database, this geographical datum may be automatically sent along with the PWV-related datum or an identifier may be used (as explained for the chronological age). The database DB may therefore be comprised of sections, each section being a database as illustrated on FIG. 5 for a specific area. When computing the score, a geographical datum of the user is then used when querying the database. Using a geographical datum is entirely seamless for the user, as the measuring device 200 or the user device 220 usually already stores a geographical datum (or may easily access such geographical datum). However, some analysis carried out with values of PWV-related data showed that the values were quite consistent between the areas. However, should it not be the case, the geographical datum may be useful to provide a more meaningful score.

. In a similar manner, ethnicity or a gender of the user may be taken into account. A process as described for the location-based vascular score similarly applies. The gender is usually already known by the user device 220 or the measuring device 200.

. Thus, devices, systems and methods for providing a vascular score with minimum invasive inputs (both physically and psychologically) is provided. One skilled in the art will appreciate that the present invention can be practiced by other than the described embodiments, which are presented for illustration and not of limitation, and the present invention is limited only by the claims appended to the description.

### References

[1] "General Cardiovascular Risk Profile for Use in Primary Care", The Framingham Heart Study, D'Agostino Sr et al. DOI: 10.1161/CIRCULATIONAHA.107.699579
[2] "Early and Supernormal Vascular Aging - Clinical Characteristics and Association With Incident Cardiovascular Events", Bruno et al., Hypertension, 2020. DOI: 10.1161/HYPERTENSIONAHA. 120.14971
[3] "Concept of Extremes in Vascular Aging - from Early Vascular Aging to Supernormal Vascular Aging", Laurent et al., Hypertension. 2019;74:218-228. DOI: 10.1161/HYPERTENSIONAHA. 119.12655.
[4] "The Performance of Vascular Age in the Assessment of Cardiovascular Risk of Patients with Rheumatoid Arthritis", Ferraz-Amaro et al, J. Clin. Med. 2020, 9, 4065 DOI: 10.3390/jcm9124065

### Clauses

. The following clauses illustrate some implementations of the disclosure.
1. A method to compute a vascular score for a user, the method comprising:
   retrieving a pulse wave velocity (PWV) related datum of the user;
   retrieving a chronological age datum of the user;
   querying a database using the retrieved PWV-related datum and the chronological age datum to return a quantile value, the database comprising:
      for a plurality of age groups, a repartition by quantiles of a plurality of PWV-related data; and
      the value of the PWV-related datum associated with each quantile;
      wherein the returned quantile is the value of the quantile related to the retrieved PWV-related datum;
   computing, using the retrieved chronological age, the returned quantile value and a predetermined law, a vascular score;
   generating for display an indication associated with the vascular score.
2. The method of item 1, wherein
   the measuring device is of a specific type and the PWV-related data of the database were obtained by measuring devices of the same specific type, such that all PWV-related data are homogeneous.
3. The method of any of items 1-2, wherein
   the vascular score is a vascular age that has a unit similar to a chronological age.
4. The method of item 3, wherein
   the computing includes adding or subtracting an age to the retrieved chronological age.
5. The method of item 4, wherein
   the maximum added or subtracted age is comprised between 2 and 40 years.
6. The method of any of items 1-5, wherein
   the predetermined law is configured so that the closer the retrieved quantile to the 0,5 quantile, the closer the computed vascular age to the chronological age.
7. The method of any of items 1-6, wherein
   the predetermined law is configured so that the highest variation of the score for quantiles above 0.5 is higher or equal than the highest variation of the score for quantiles below 0.5.
8. The method of any of items 1-7, wherein
   the predetermined law is a piecewise function, wherein each piece of the function applies at least on a specific interval of retrieved quantiles and is defined with a set of parameters, the values of which depend on the intervals of quantiles, and at least two sets of parameters of two pieces of the function have different values.
9. The method of item 8, wherein
   a boundary of an interval of the retrieved quantiles for the piecewise function is comprised between [0.1; 0.4] and a boundary of another interval of the retrieved quantiles for the piecewise function is comprised between [0.6; 0.9].
10. The method of any of items 1-9, wherein
   the predetermined law is a piecewise function, wherein each piece of the function applies at least on a specific age group and is defined with a set of parameters, the values of which depend on the age group, and at least two sets of parameters of two pieces of the function have different values.
11. The method of any of items 1-10, wherein
   the database comprises at least twenty quantiles.
12. The method of any of items 1-11, wherein
   the indication generated for display is an alphanumeric indication telling the user he or she is older or younger than his or her chronological age.
13. The method of any of items 1-12, wherein
   any PWV-related datum is an aortic-leg PWV-related datum.
14. The method of any of items 1-13, wherein
   no regression is performed in the database and computing the vascular score involves no regression.
15. The method of any of items 1-14, wherein
   the indication associated with the vascular score is the vascular score.
16. A device for computing a vascular score for a user, the device comprising control circuitry with a processor and a storage storing a database, the control circuitry being configured to:
   retrieve a pulse wave velocity (PWV) related datum of the user;
   retrieve a chronological age datum of the user;
   query a database using the retrieved PWV-related datum and the chronological age datum to return a quantile value, the database comprising:
      for a plurality of age groups, a repartition by quantiles of a plurality of PWV-related data; and
      the value of the PWV-related datum associated with each quantile,
      wherein the returned quantile is the value of the quantile related to the retrieved PWV-related datum;
   compute, using the retrieved chronological age, the returned quantile value and a predetermined law, a vascular score;
   generate for display an indication associated with the vascular score.
17.The device of item 16, wherein control circuitry is further configured to display the indication associated with the vascular score.
18. The device of any of items 16-17, wherein the device is a weighing scale and the PWV-related datum is determined by the weighing scale.
19. The device of any of items 16-18, where control circuitry is further configured to perform any of the methods of items 1-15.
20. A system comprising a fleet of devices according to any of items 16-19 and a server, wherein the server is configured to:
   retrieve, from the devices, a plurality of PWV-related data and retrieve chronological ages associated with the PWV-related data,
   generate the database.
21. A non-transitory computer-readable medium having instructions encoded thereon that when executed by control circuitry cause the control circuitry to carry out the method of any of items 1-15.
22. A computer program comprising instructions which, when the program is executed by a computer, cause the computer to carry out the method of any of items 1-15.
23. A method to compute a vascular score for a user, the method comprising:
   retrieving a pulse wave velocity (PWV) related datum of the user;
   retrieving a chronological age datum of the user;
   querying a database using the chronological age datum to return a median of PWV-related data associated with the chronological age, the database comprising, for each of a plurality of age groups, a median of the PWV-related data;
   computing, using the retrieved chronological age, the retrieved PWV-related datum, the median PWV-related value, and a predetermined law, a vascular score;
   generating for display, an indication associated with the vascular score.
24. The method of item 23, wherein
   the measuring device is of a specific type and the PWV-related data of the database were obtained by measuring devices of the same specific type, such that all PWV-related data are homogeneous.
25. The method of any of items 23-24, wherein
   the vascular score is a vascular age that has a unit similar to a chronological age.
26. The method of item 25, wherein
   the computing includes adding or subtracting an age to the retrieved chronological age.
27. The method of item 26, wherein
   the maximum added or subtracted age is comprised between 2 and 40 years.
28. The method of any of items 23-27, wherein
   the predetermined law is configured so that the closer the PWV to the median, the closer the computed vascular age to the chronological age.
29. The method of any of items 23-28, wherein
   the predetermined law is configured so that the highest variation of the score for PWV-related data above the median is higher or equal than the highest variation of the score for PWV-related data below the median.
30. The method of any of items 23-29, wherein
   the predetermined law is a piecewise function, wherein each piece of the function:
   applies at least on a specific interval of a comparison between the retrieved PWV-related datum and the median; and
   is defined with a set of parameters, the values of which depend on said intervals, and at least two sets of parameters of two pieces of the function have different values.
31. The method of item 30, wherein
   a boundary of an interval for the piecewise function is comprised between [0.8; 0.9] and a boundary of another interval of the retrieved quantiles for the piecewise function is comprised between [1.1; 1.2], wherein the comparison is a ratio of the retrieved PWV-related datum over the median.
32. The method of any of items 23-31, wherein
   the predetermined law is a piecewise function, wherein each piece of the function applies at least on a specific age group and is defined with a set of parameters, the values of which depend on the age group, and at least two sets of parameters of two pieces of the function have different values.
33. The method of any of items 23-32, wherein
   the indication generated for display is an alphanumeric indication telling the user he or she is older or younger than his or her chronological age.
34. The method of any of items 23-33, wherein
   any PWV-related datum is an aortic-leg PWV-related datum.
35. The method of any of items 23-34, wherein
   no regression is performed in the database and computing the vascular score involves no regression.
36. The method of any of items 23-35, wherein
   the measuring device is of a specific type and the PWV-related data of the database were obtained by measuring devices of the same specific type, such that all PWV-related data are homogeneous.
37. The method of any of items 23-36, wherein
   the indication associated with the vascular score is the vascular score.
38. A device for computing a vascular score for a user, the device comprising control circuitry with a processor and a storage storing a database, the control circuitry being configured to:
   retrieve a pulse wave velocity (PWV) related datum of the user;
   retrieve a chronological age datum of the user;
   query a database using the chronological age datum to return a median of PWV-related data associated with the chronological age, the database comprising, for each of a plurality of age groups, a median of the PWV-related data;
   compute, using the retrieved chronological age, the retrieved PWV-related datum, the median PWV-related value, and a predetermined law, a vascular score;
   generate for display, an indication associated with the vascular score.
39. The device of item 38, wherein control circuitry is further configured to perform any of the methods of items 23-37.
40. The device of any of items 38-39, wherein control circuitry is further configured to display an indication associated with the vascular score.
41. The device of any of items 38-40, wherein the device is a weighing scale and the PWV-related datum is determined by the weighing scale.
42. A non-transitory computer-readable medium having instructions encoded thereon that when executed by control circuitry cause the control circuitry to carry out the method of any of items 23-37.
43. A computer program comprising instructions which, when the program is executed by a computer, cause the computer to carry out the method of any of items 23-37.

## Claims

1. A method to compute a vascular score for a user, the method comprising:
retrieving (602) a pulse wave velocity (PWV) related datum of the user;
retrieving (604) a chronological age datum of the user;
querying (606) a database (DB, 500) using the retrieved PWV-related datum and the chronological age datum to return a quantile value, the database comprising:
for a plurality of age groups, a repartition by quantiles of a plurality of PWV-related data; and
the value of the PWV-related datum associated with each quantile;
wherein the returned quantile is the value of the quantile related to the retrieved PWV-related datum;
computing (608), using the retrieved chronological age, the returned quantile value and a predetermined law, a vascular score;
generating for display (612) an indication associated with the vascular score.

2. The method of claim 1, wherein
the PWV related datum is determined using a measuring device that is of a specific type and the PWV-related data of the database were obtained by measuring devices of the same specific type, such that all PWV-related data are homogeneous.

3. The method of any of claims 1-2, wherein
the vascular score is a vascular age that has a unit similar to a chronological age.

4. The method of any claims 1-3, wherein
the computing includes adding or subtracting an age to the retrieved chronological age, for example the maximum added or subtracted age is comprised between 2 and 40 years.

5. The method of any of claims 1-4, wherein
the predetermined law is configured so that the closer the retrieved quantile to the 0,5 quantile, the closer the computed vascular age to the chronological age.

6. The method of any of claims 1-5, wherein
the predetermined law is configured so that the highest variation of the score for quantiles above 0.5 is higher or equal than the highest variation of the score for quantiles below 0.5.

7. The method of any of claims 1-6, wherein
the predetermined law is a piecewise function, wherein each piece of the function applies at least on a specific interval of retrieved quantiles and is defined with a set of parameters, the values of which depend on the intervals of quantiles, and at least two sets of parameters of two pieces of the function have different values.

8. The method of claim 7, wherein
a boundary of an interval of the retrieved quantiles for the piecewise function is comprised between [0.1; 0.4] and a boundary of another interval of the retrieved quantiles for the piecewise function is comprised between [0.6; 0.9].

9. The method of any of claims 1-8, wherein
the predetermined law is a piecewise function, wherein each piece of the function applies at least on a specific age group and is defined with a set of parameters, the values of which depend on the age group, and at least two sets of parameters of two pieces of the function have different values.

10. The method of any of claims 1-9-, wherein
the database comprises at least twenty quantiles.

11. The method of any of claims 1-10, wherein
the indication generated for display is an alphanumeric indication telling the user he or she is older or younger than his or her chronological age.

12. The method of any of claims 1-11, wherein
no regression is performed in the database and computing the vascular score involves no regression.

13. A device (103, 106, 200) for computing a vascular score for a user, the device comprising control circuitry (202) with a processor (212) and a storage (214) storing a database, the control circuitry being configured to perform a method according to any claims 1-15.

14. A system comprising a fleet (102) of devices (103, 106) according to claim 13 and a server (101), wherein the server is configured to:
retrieve, from the devices, a plurality of PWV-related data and retrieve chronological ages associated with the PWV-related data,
generate the database (DB, 500).

15. A non-transitory computer-readable medium having instructions encoded thereon that when executed by control circuitry cause the control circuitry to perform a method according to any claims 1-13.
